Europäisches Patentamt

(19) European Patent Office

Office européen des brevets

(11) Publication number: **0 185 126**
**B1**

(12) **EUROPEAN PATENT SPECIFICATION**

(45) Date of publication of patent specification: **15.11.89**

(21) Application number: **84810601.9**

(22) Date of filing: **10.12.84**

(51) Int. Cl.⁴: **G 01 N 21/03,** G 01 N 21/75,
G 01 N 33/552

(54) Optical test probe and apparatus for optically determining species in solution.

(43) Date of publication of application:
**25.06.86 Bulletin 86/26**

(45) Publication of the grant of the patent:
**15.11.89 Bulletin 89/46**

(84) Designated Contracting States:
**CH DE FR GB IT LI**

(56) References cited:
**WO-A-83/01112**
**US-A-3 939 350**
**US-A-4 050 895**
**US-A-4 169 676**

**ANALYTICAL CHEMISTRY, vol. 45, no. 4, April
1973, pages 687-691; N.J. HARRICK et al.:
"Multiple internal reflection fluorescence
spectrometry"**

(73) Proprietor: **PRUTEC LIMITED**
**142 Holborn Bars**
**London EC1N 2NH (GB)**

(72) Inventor: **Sutherland, Ranald**
**23, rue de la Filature**
**CH-1227 Carouge (CH)**
Inventor: **Dähne, Claus**
**103, avenue du Bois de la Chapelle**
**Ch-1213 Onex (CH)**
Inventor: **Revillet, Georges**
**20, chemin Francois Chavaz**
**CH-1213 Onex (CH)**

(74) Representative: **Dousse, Blasco et al**
**7, route de Drize**
**CH-1227 Carouge/Genève (CH)**

Courier Press, Leamington Spa, England.

EP 0 185 126 B1

# EP 0 185 126 B1

**Description**

The present invention concerns an optical test probe including an analytical cuvette and light guiding means and an apparatus for optically determining species in solution, more especially for determining bioactive species by reactions of the immunoassay type.

Analytical apparatuses comprising optical fiber probes which can optically monitor the adsorption of chemical species on the fiber core are known. This technique is based on the immersion of a lit optical waveguide, for instance an optical fiber without cladding, in a test solution the refractive index of which is lower than that of the fiber core, whereby an interaction takes place between the evanescent wave component of the signal travelling along the waveguide and some species in solution to be determined. This approach is particularly interesting for monitoring events in the reaction space in close vicinity to the fiber, i.e. within reach of the evanescent wave component (a few tenths or hundredths of angströms), this being in the case of tests based on the reaction of a first partner in a complexation reaction, this partner being adsorbed or attached on the probe surface, with a second partner dissolved in the sample solution.

Apparatuses suitable for such types of measurements have been recently disclosed in the following references WO 84/00817; USP—4,447,546 (Hirschfeld et al); GB 2,103,786 (ICI); J. D. Andrade et al. Applied optics 23 (11) 1984, 1812—1815; WO—A—8100912 (Buckles); US—A—4,050,895 (Hardy et al); US—A—3,939,350 (Kronick et al).

Recently, there has been disclosed (see EP—A—75353) an apparatus for measuring parameters in a reaction of an analyte with a specific reactant thereto, said reaction occurring on the surface of a waveguide, e.g. a piece of optical fiber, and causing detectable changes to the optical properties thereof, which comprises a light source, means to inject a signal from that source into the input of said waveguide, detecting means to detect the light signal having undergone changes when travelling therethrough and emerging therefrom and converting it to an electric signal, and means for processing said signal into useful data pertaining to said reaction. This apparatus comprises:

a) A waveguide, the main part of which passes through a container or cuvette for holding a liquid analyte to be determined; the exposed surface of the waveguide immersed in the liquid is coated, before operation, with a thin film of a specific complexing reagent of the species dissolved in the liquid and which should be determined. The assembly of the waveguide and the cuvette constitutes the test probe of the apparatus.

b) A light source, a collimating lens, an annular aperture and a focusing lens for injecting into the waveguide a light beam originating from the source and directed by the focusing lens at a selected angle to ensure propagation of the beam by multiple reflections in the probe fiber.

c) The disclosed apparatus further comprises a main detector for transforming the exit light signal from the output end of the core into an electric signal, amplifying and computing circuits to process the signal from the detector and, finally, a display device providing the desired read-out output.

Although the previously disclosed test probe for performing the chemical reaction to be monitored has been operated satisfactorily in the past, it was found desirable to provide other systems which can be set-up faster, easier to operate and which can provide information, more or less simultaneously, on more than one parameter of the analytical solution.

The present probe, as defined in claim 1 is an accomplishment of the aforementioned wishes. One embodiment of this probe and an apparatus in which it is incorporated as well as modifications thereof will now be described with reference to the annexed drawing.

Fig. 1 is a schematic top view of an embodiment of an analytical apparatus involving a test probe with two waveguide elements.

Fig. 2 is a schematic view of a variant of the embodiment of Fig. 1.

Fig. 3 is a schematic view of still another embodiment.

Fig. 4 is a diagram explaining the propagation of totally reflected light in a medium (waveguide) of refractive index $n_1$ greater than $n_2$, the refractive index of another medium (analyte) with which the waveguide is in contact.

Fig. 5 is appendant to Fig. 4 and schematically represents the penetration of the evanescent wave component in the rarer medium (analyte).

Fig. 6 is a schematized representation of the phenomena occurring during an analysis using a waveguide according to the invention.

Fig. 7 is a diagram showing response curves in performing an analysis according to the aforementioned representation.

Fig. 8 is a diagram with a typical response curve in the analysis of $HbA_{1c}$, a blood component, in the presence of hemoglobin.

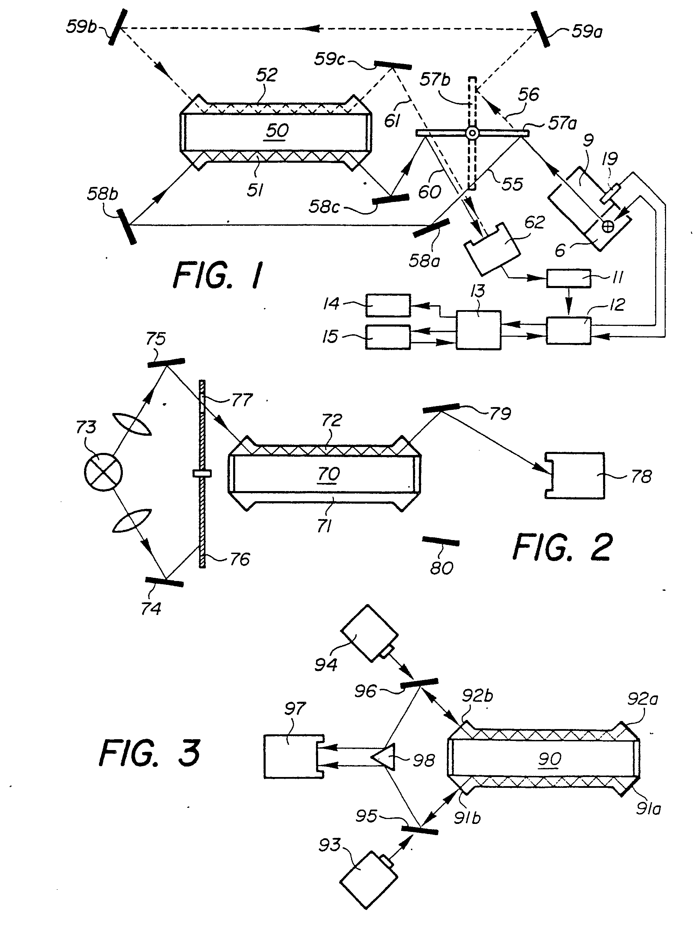

The apparatus of which the essential optical components are schematized on Fig. 1 comprises a dual-element waveguide cell 50 of which the main walls 51 and 52 constituting the two light guiding elements transport exciting signals originating from a source 6 and whose inner walls are in contact with the analyte solution contained in cell 50. The especially shaped light conductive walls of the cuvette can be provided by usual means, e.g. by molding with a transparent plastic, for instance lucite or they can be made of glass, preferably optical grade. The width of the cuvette, i.e. the distance between walls 51 and 52 is any compatible with the holding of a definite amount of analyte; this width can be a few mm or a fraction of a mm (e.g. 10 to 1000 μm) in which case the cuvette can also act as a sampling means due to the capillary effect provided by walls arranged very near to each other.

A beam of light originating from a source 6 is alternatively split into beams 55 and 56 by a rotating chopper mirror 57a, b. On Fig. 1, this mirror 57 has been represented in two positions, i.e. one instant position corresponds to numeral 57a and another position (at about right angle to the first one) by numeral 57b. It can be readily seen that, depending upon the position of the mirror 57, the original beam is either reflected into beam 55 or into beam 56. Thus, the light from the source 6 is alternatively injected in either portion 51 or 52 of the dual waveguide cell 50 by means of either one of a series of mirrors 58a, b, c and 59a, b and c, respectively. The output light, respectively 60 and 61, from either part of the waveguide is then collected on a detector 62.

The remaining components of this embodiment comprise a monochromator 9 and electronic elements with data-acquisition and processing microcomputer, including a photomultiplier detector 62, a preamplifier 11, a light source microcomputerized control system 12, a microprocessor 13, a printer 14, and a memory (floppy disc) 15.

The light source 6 used preferably was a xenon flash lamp (E. G. & G., Salem, MA) and the monochromator was preferably equipped with a concave holographic grating (Jobin-Yvon, Paris, France) to permit a resolution of 5 nm. The flash lamp of the source 6 was controlled by the microcomputer 12. To inject the samples to the cell 50 a programmable automatic pipette (Microlab-P; Hamilton Bonaduz AG, Bonaduz, Switzerland) was used preferably. The detector 62 comprised a photomultiplier tube (R928; Hamamatsu, Tokyo, Japan) placed at the waveguide output to monitor the change in light intensity directly. Signals from the photomultiplier tube were amplified (11), integrated during the flash time (12) and converted by a standard 12-bit analog/digital converter (not shown) into digital format. The in-house microcomputer 12 performed fast signal averaging, and all data were adjusted for variation in flash lamp intensity by reference to a photodiode 19 placed in the monochromator. The signals were transmitted to an Apple II microcomputer 13 for display and storage.

In a further embodiment, (see Fig. 2), the apparatus comprises a dual-waveguide cell 70 identical with the cell of the previous embodiment, i.e. having walls 71 and 72 acting as the two independent waveguide elements and operating similarly as will be seen.

The apparatus comprises a light source 73, the output of which is focused on either side by means of lenses and mirrors, respectively 74 and 75, on the input side of guide elements 71 and 72, respectively. A chopper disc 76 with a window hole 77 acts to alternatively distribute the exciting light into elements 71 and 72. The output signals from the waveguide are then directed to a detector 78 by means of mirrors 79 and 80.

In both embodiments depicted on Figs. 1 and 2, one of the waveguide elements (51, 71) is coated with an antibody specific to one component to be measured in the analyte by a complexation reaction (as disclosed hereintofore) while the second element (52, 72) is left uncoated. Herein, *uncoated* refers to a surface without antibody. However the protein adsorption sites on this surface are usually blocked by adsorbing a non-binding protein (e.g. bovine serum albumin BSA) to the surface. Therefore during analysis, the signal at the output of the uncoated area reflects the interaction of the exciting beam with the bulk of the analyte. However, simultaneously or nearly simultaneously, the signal emerging from the coated side of the guide provides the required information on the component being bound by the specific reactant coated on the inner surface of this side of the cell. This will be illustrated in more detail with reference to the examples in this application. Suffice to say now that this kind of waveguide system (dual type) allows to gather the two types of information from separate elements of the waveguide. Evidently the detector output signal in this embodiment is thereafter processed and converted into read-out data as disclosed in connection with the previous embodiment.

Another variant embodiment is represented on Fig. 3. In this variant, a dual waveguide cell 90 of the same general configuration as the aforementioned cells 50 and 70 is used with the difference that ends 91a and 92a are actually made reflective, for instance by metallizing (silver or aluminum) like in the case of a mirror. Therefore, the other ends 91b and 92b, respectively, of the waveguide light conductive elements act simultaneously as the input and output ends. This is illustrated by the paths of the exciting light beams provided by two sources 93 and 94 which are directed into ends 91b and 92b, respectively, after crossing beam-splitters 95 and 96, respectively. Thus, the light which penetrates through ends 91b and 92b travels through the guide first in a forward direction and then backwards after being reflected from ends 91a and 92a. This configuration enables that the interaction capacity of the exciting light with the analytes be actually doubled as compared with the previously disclosed embodiments. This variant further comprises a detector 97 for collecting the backward signals exiting from 91b and 92b and directed thereto by means of beam splitters 95 and 96 and a prism-like mirror 98. Sources 93 and 94 are alternatingly synchronized by means known in the art so that signal pulses exiting from the waveguide ends 91b and 92b do not simultaneously fall on the detector 97. Naturally, other types of cuvette-waveguide probes with more than two waveguide elements are also possible, for instance cuvettes containing optical fiber waveguides in addition to the opposite facing walls thereof working as waveguides. Therefore the total number of independent waveguides elements operating together in the unit being larger than 2, a corresponding number of parameters of the solution can be investigated simultaneously (or about simultaneously).

The use of the afore-disclosed apparatuses can be illustrated by the analysis of blood samples as provided hereinafter and also in co-pending application EP—A—184 600.

Indeed, the direct determinations in blood samples of hemoglobin and various other hemoglobin factors such as glycosylated hemoglobin, this being, if desired, relative to total hemoglobin in this sample constitute very important medical tests. It is known that glycosylated hemoglobins ($HbA_{1a}$, $A_{1b}$ and $A_{1c}$) are important factors in the diagnosis and monitoring of patients with diabetes. The determination of the content of $HbA_{1c}$ (which amounts to about 80% of total glycosylated hemoglobins ($HbA_1$)) relative to total hemoglobin (i.e. $HbA_0$, non-glycosylated hemoglobin and $HbA_1$) is particularly important with regard to that disease.

Hemoglobin $A_{1c}$ is a glycohemoglobin with an amino acid structure which is identical to that of $HbA_0$; the important difference is the presence of 1 - amino - 1 - deoxy - fructose attached in the 2,3 - diphosphoglycerate pocket to the N-terminal valine in the beta-chain of $HbA_{1c}$. The modification of $HbA_0$ to $HbA_{1c}$ is a continuous non-enzymatic post-translation process, the rate of which is a function of the blood glucose concentration. Glycosylation occurs as a two step process. Firstly, the open aldehydic form of glucose reacts with the terminal amino group of the beta-chains of Hb to form a Schiff base. Secondly, the Schiff base then undergoes an Amadori rearrangement to form $HbA_{1c}$. The intermediate Schiff base is unstable with a 60-fold greater tendency to dissociate (to the free sugar plus protein) than the stable ketoamine of $HbA_{1c}$. As only a small fraction of blood glucose is in the open aldehydic form (approximately $10^{-6}$%) and the rate of ketoamine formation is slow (although effectively irreversible), the formation of $HbA_{1c}$ is an indication of long term blood glucose concentration. Over the 120 days life-span of the human red blood cell, the number of glycosylated Hb molecules increases proportionally to the mean blood glucose concentration. The relationship between the mean plasma glucose and $HbA_{1c}$ concentration is unique in that a single $HbA_{1c}$ extinction measurement provides a retrospective assessment of blood glucose control over the preceding 6 to 8 weeks. It is generally accepted that $HbA_{1c}$ measurements are a very useful tool in monitoring diseases of carbohydrate metabolisms, specifically diabetes mellitus. Diabetics have high long-term blood sugar levels and this is reflected in their $HbA_{1c}$ levels. Normal adults have about 3—6% of their total hemoglobin as $HbA_{1c}$ whereas the range in juvenile and maturity onset diabetics is 6—15% as $HbA_{1c}$. A similar increase in $HbA_{1c}$ concentration has been noted in mice with genetic and chemically induced diabetes and in pancreatectomized dogs.

Among the several methods which exist for determining glycosylated Hb in blood, $HbA_1$ and in particular $HbA_{1c}$ measurements have now become a method of choice for monitoring the treatment of diabetics (L. Jovanovic et al., American J. of Medicine (1981) 70, 331; D. E. Goldstein et al., Diabetes (1982) 31, 70; K. H. Gabboy et al., J. of Clinical Endocrinology and Metabolism (1977) 44, 859; B. Gonen et al., Diabetologia (1978) 15, 1; C. M. Peterson, Diabetes (1982) 31, 1). Also, the following patent documents can be usefully mentioned: US—A—4,247,553; GB—A—1,580,318; US—A—4,222,836; US—4,372,747; US—4,200,435; US—4,341,635. These methods can be readily classified by the mechanism used to separate glycosylated Hb from non-glycosylated Hb. For instance ion-exchange chromatography was used early and is still the most common method (H. G. Kunkel et al., Science (1955) 122, 288). Although such ion-exchange technique is currently the only available method which specifically measures $HbA_{1c}$, it has a number of limitations of which temperature and pH sensitivity are the most important. Ion-exchange also is subject to interferences as labile glycosylated Hb (pre-$HbA_{1c}$) must be removed prior to assay and both fetal Hb (HbF) and Sickle Cell Hb (HbS) interfere with the results.

Other techniques involve agar gel electrophoresis (L. Menard et al., Clinical Chemistry (1980) 26, 1598), isoelectric focusing (K. M. Spicer et al., Diabetes (1978) 27, 384), colorimetry, e.g. with thiobarbituric acid (R. Fluckiger et al., Febs Letters (1976) 71, 356) and affinity chromatography (V. Bouriotis et al., Diabetologia (1981) 21, 579). Only one type radioimmunoassay has been reported (J. Javid et al., British J. of Haematology (1978) 38, 329) which was slow (more than 3 days to work) and technically complex as requiring the preparations of radiolabelled $HbA_{1c}$. Although the methods of the prior art have merits, there is still a need for methods providing quick results (less than about 15 minutes), requiring less skilled operators and less costly to be undertaken on a routine basis. Current art methods are slow (typically more than 1 hour results), technically complicated (require more than five pipetting manipulation steps) and unsuited to testing outside a laboratory environment. Further, present methods require that total hemoglobin be ascertained separately from the glycosylated factors and it was desirable that both analytical data could be ascertained substantially together and correlated without delay.

The method which can be performed with the apparatus disclosed in the present invention remedies the inconvenience of prior art methods and enables to determine about simultaneously more than one parameter in the blood; it further offers the advantage of directly providing, if desired, the percent of one parameter, e.g. a glycosylated factor or other hemoglobin factors relative to another parameter, e.g. total hemoglobin.

This illustrative method allows for the separate determination of Hb-$A_{1c}$, $A_{1a}$ or $A_{1b}$ provided antibodies specific to any of such species are available in purified form. Otherwise, using less specific antibodies, the present method allows for the combined determination of two or more blood factors taken together, i.e. all glycosylated Hb relative to total Hb for instance. Of course, the method also provides for the determination of blood factors other than the ones hereabove if corresponding reagents specific to said factors in complex formation reactions are available (e.g. HbF, HbS or other human hemoglobin variants).

The method does not concern the obtention or preparation of such specifically reactive complex moieties (monoclonal or polyclonal antibodies) but it concerns their use as coating materials in the preparation of the active waveguides to be contacted with the blood sample to be analyzed with the assembly of the invention.

The optical technique used here relates, as heretofore mentioned, mainly to light adsorption, i.e. there is an interaction of the evanescent component of the wave transported in one operative element of the guide with the molecules, first in the ambient liquid and, second, with the Hb-antibody complex which starts building up on the guide in form of a layer due to the reaction of the blood factor to be determined with the specific complex moiety (antibody) previously coated on the surface of another operatively distinct element of the waveguide. The depth of interaction of the evanescent light component in the corresponding element is substantially limited to the thickness of the layer of the complex so the optical response to that build-up is independent of the bulk adsorption due to the blood itself and the two effects can be easily distinguished without sophisticated techniques for decoding the signals originating from one or the other effect.

Hb derivatives have characteristic adsorption spectra dependent on their chemical state. Hence, any of the usual adsorptiometric techniques are equally applicable for implementing the invention (L. Tentori et al, Hemoglobin, in Methods in Enzymology (1981), vol. 76, 707—732, Academic Press, New-York). Included are the cyanomethemoglobin method and single or multi-wavelength absorptionmetric assays, preferably in the range 400 to 600 nm, specifically 400—420 nm and 550—600 nm. Also included are such isobestic point methods where the absorption by the Hb molecule is independent of the degree of oxygen saturation.

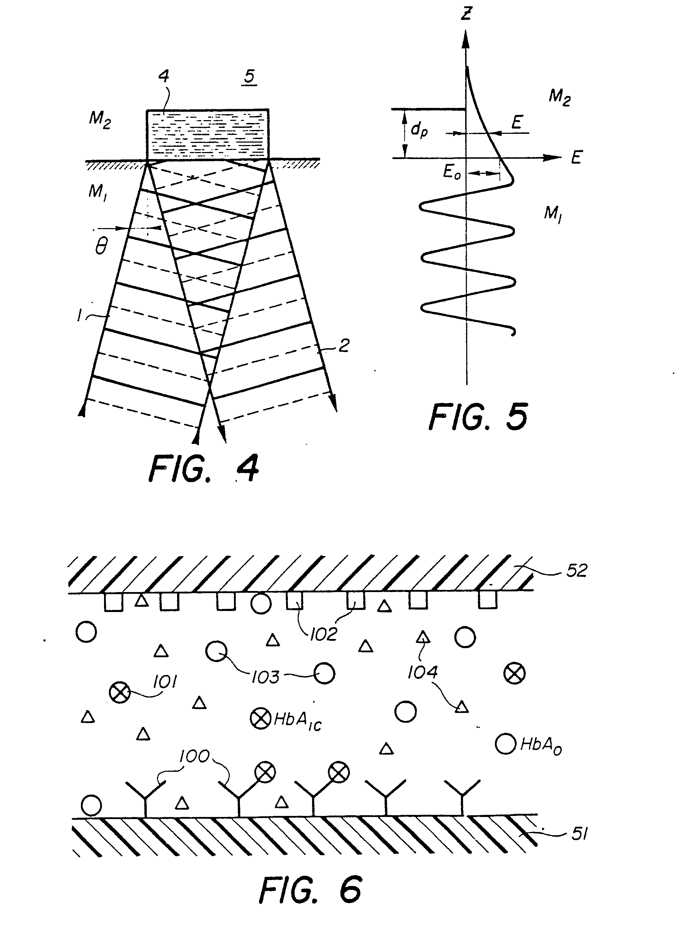

The operation of the present test proceeds according to the following: when a light beam 1 strikes with an angle $\theta$ the interface between two transparent media $n_1$ and $n_2$ (Fig. 4) striking from the medium $n_1$ with the greater refractive index $(n_1 > n_2)$, total internal reflection occurs (N. J. Harrick, Internal Reflexion Spectroscopy, Wiley Interscience, New-York (1967) when the angle of reflection $\theta$ is larger than a certain value $\theta_c$ called the critical angle given by the equation:

$$\theta_c = \sin^{-1}(n_2/n_1) \tag{1}$$

The reflected beam is indicated by numeral 2. In this case the evanescent wave penetrates a distance $(d_p)$ of the order of a fraction of a wavelength beyond the reflecting surface into the rarer medium of refractive index $n_2$. According to Maxwell's equations a standing sinusoidal wave, perpendicular to the reflecting surface, is established in the denser medium (Fig. 5). Although there is no net energy flow into the non absorbing, rarer medium, there is an evanescent, nonpropagating field in that medium, the electric field amplitude (E) of which is largest at the surface interface (Eo) and decays exponentially with the distance (Z) from the surface according to:

$$E = E_o \cdot \exp\left(-Z/d_p\right) \tag{2}$$

The depth of penetration $(d_p)$, defined as the distance required for the electric field amplitude to fall to exp (−1) of its value at the surface, is given by:

$$d_p = \frac{\lambda/n_1}{2\pi(\sin^2\theta - (n_2/n_1)^2)^{1/2}} \tag{3}$$

Starting from 90°C, as $\theta$ approaches $\theta_c$, $d_p$ becomes infinitely large, and at a fixed angle, increases with closer index matching (i.e., as $n_2/n_1 \rightarrow 1$). Also, because $d_p$ is proportional to wavelength, it is greater at longer wavelengths.

Thus, by an appropriate choice of the refractive index $n_1$ of the transparent waveguide, of the incident angle, and of the wavelength, one can select a $d_p$ to control optical interaction mainly with substances 4 close or at given distance from the interface and minimally with substances 5 beyond said distance. In the present embodiment, the denser medium can be constituted by the wall of the optical cell or cuvette of the invention made of plastic or glass ($n_1$ being of the order of 1.40 to 1.60) and the rarer medium is the aqueous blood sample ($n_2 = 1.34$); $\theta$ is controllably variable so that when $\lambda$ is a selected visible wavelength, $d_p$ can be varied from about 20 to 300 nm. In the unit of the invention, the materials used for the different waveguide elements, e.g. the walls of the cuvette can be different in nature, i.e. have different refractive index $(n_1)$; therefore the depth of penetration of the evanescent wave component travelling in one element can be different from that in another element, with the result that the interaction between the exciting signal and the analytical species to be determined can involve regions at different depths within the solution to be analyzed.

The number of reflections (N) in the waveguide is a function of the length (L) and thickness (T) of waveguide and angle of incidence $(\theta)$:

$$N = L/T \cdot \cotg\ \theta \tag{4}$$

In the waveguides used here the total number of reflections for a discrete light beam varied from about 30 to 50.

Fig. 6 is a schematic illustration at the molecular level of the phemomena taking place during analysis in a cell of the dual-waveguide type as disclosed previously. In Fig. 6 areas marked 51 and 52 correspond for instance to the waveguide elements 51 and 52 depicted on Fig. 1. The area intermediate between areas 51 and 52 represents schematically an analyte medium with species dissolved therein and species attached to the inside walls of elements 51 and 52. Element 51 is pictured to have deposited thereon antibodies 100 specific to $HbA_{1c}$ entities labelled 101. Some of these $HbA_{1c}$ molecules are shown after complexation with the specific antibody 100, others are still free. The other surface (i.e. the surface of element 52) is shown coated with blocking agents 102 (for instance, bovine serum albumin) said agents being intended to minimize the possible affinity of the bare wall to other species in solution, for instance $HbA_0$ 103 and other proteins of any type 104.

Thus, during analysis, non-specific binding of Hb to surface 52 is prevented (or at least strongly minimized) which makes it possible, by using an angle θ of suitable value, to measure the bulk hemoglobin by the interaction of the evanescent wave component of the signal travelling in 52 with the analyte solution at depths beyond that of the blocking coating deposited on the surface.

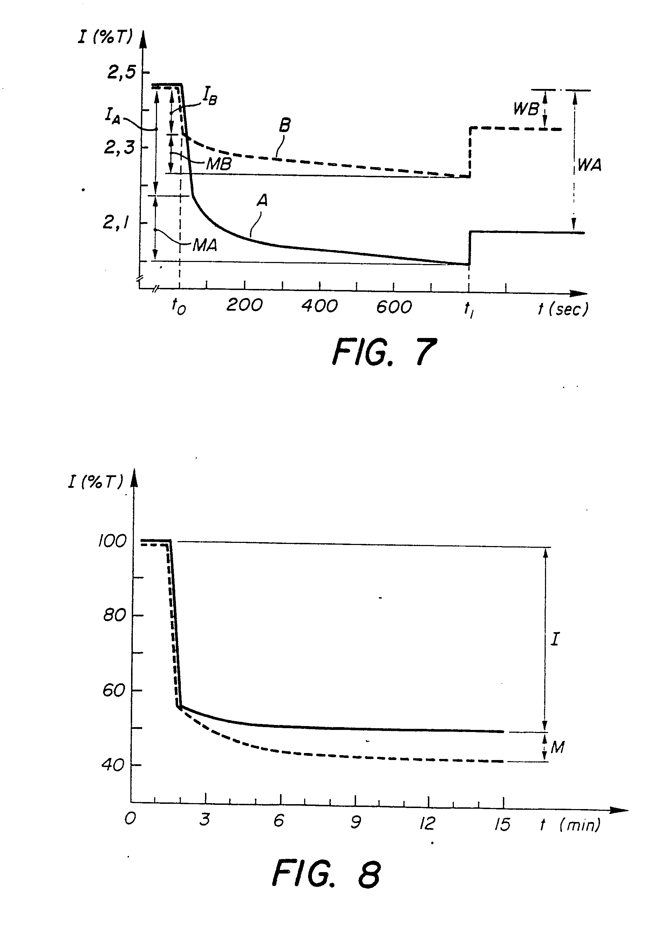

In contrast, a complexation reaction occurs on surface 51 between the antibody molecules 100 coated thereon and the $HbA_{1c}$ (AG) molecules in the analyte solution. This reaction although rapid is not instantaneous; therefore a layer of complex progressively builds up on surface 51 with consecutive corresponding interaction with the light component travelling in that element of the waveguide, this resulting in the production of response curves of the A or B type depicted in Fig. 7 (see the examples that follow).

In order to practically carry out the tests, the glass cuvettes were cleaned by consecutive immersion in concentrated sulfuric acid and distilled water, ethanol, and acetone, using standard slide-staining glasswave. Other non glass waveguides were cleaned in ethanol ultrasonically. The waveguides were contacted with the various antibody solutions. Antibodies were either physically adsorbed to the surface of the waveguides or covalently coupled. Adsorption was carried out by incubating cleaned waveguides with solutions of antibody (5 μg of protein/ml of 0.05 mol/l Tris Hcc buffer, pH 7.0) for 4 hours. Unadsorbed proteins were washed away with saline and residual protein binding sites blocked by incubation of the antibody-coated waveguides with bovine serum albumin (1.0% by weight in TRIS Buffer). The method of coupling was essentially that of Weetall, involving 3 - aminopropyl - triethoxysilane APTS (Immobilized Biochemicals and Affinity Chromatography, R. G. Dunlop, Plenum Press, New-York, p. 191—212) in an acid aqueous silanization environment. (Immobilized Enzymes, Antigents, Antibodies and Peptides: Preparation and Chromatography, 1: Enzymology, H. A. Weetall, Marcel Dekker Inc. New-York 1975, p. 1—48).

In general, we reacted waveguides with APTS (0.4 mol/l) for 3 hours at 80°C. We then heated the waveguides at 80—120°C, depending on the materials involved for 2 hours, then let them soak in glutaraldehyde (0.5 mol/l) in phosphate buffer (0.1 mol/l, pH 6.8) for 90 min at ambient temperature. The "activated" waveguides were then reacted with antiserum Ab (5 mg of protein per milliliter of phosphate buffer) for 24 hours at 4°C. After washing the antibody-coupled waveguides in phosphate buffer, we stored them at 4°C in isotonic saline (0.14 mol/l, containing sodium azide, 8 mmol/l). Measurements of protein (Anal. Biochem 51, 654—655 (1973)) before and after the coupling demonstrated protein uptakes of approximately 1 μg/cm² of quartz.

Example 1
Measurement of hemoglobin in the presence of foreign hemoglobin

Solution samples were prepared based on avian hemoglobin (pigeon) and containing variable proportions of human hemoglobin to be measured. The total of both hemoglobins was always 5 mg/ml in one series of samples (A) and the proportions of human hemoglobin are given in the Table below. In another series of samples (B) the total hemoglobin concentration was about doubled. A dual waveguide of the type shown in Figs. 1 and 2 was used, one of the surfaces (e.g. 51) being coated with antibody to human IgG. The other surface (52) was blocked with bovine serum albumin as usual.

Upon doing the measurements a sharp dip (I) corresponding to transmission loss was observed in all cases; then the further drop in transmission (M) (see Fig. 7) was recorded after an interval of 10 min. In the case of the sample containing only avian hemoglobin, no further change during the 10 min interval was observed. The results are summarized below. The subscripts A and B used in Fig. 7 correspond to samples of different concentrations of total hemoglobin.

| Human Hb in avian Hb (%) | Transmission (%) (total hemoglobin) | (M) |
|---|---|---|
| 0 | 75.3 | 0 |
| 1 | 74.9 | 0.4 |
| 2 | 74.4 | 0.9 |
| 10 | 72.0 | 3.3 |
| 20 | 68.3 | 7.0 |

Thus the value recorded for the first initial dip I can be correlated with the total hemoglobin present while the values (M) observed after the 10 min reaction period and corresponding to the binding of the human hemoglobin factor to the antibody coated on surface 51 can be correlated with the human hemoglobin content of the sample and its ratio to total hemoglobin. Standard curves were made from the above data by recording on an automatic recorder coupled to the apparatus used in this example. Such curves were thereafter used as comparative data for determining unknown mixtures of human hemoglobin in avian hemoglobin. In Fig. 7, the values W ($W_A$ and $W_B$) correspond to the responses measured after the cell was washed thoroughly with assay buffer (at $t_1$) which removed all unbound material. The W values can also be correlated, if desired, with the above analytical parameters of the solutions and used, as standards, for the analysis of unknowns.

Example 2

Measurement of glycosylated hemoglobin ($HbA_{1c}$) in the presence of hemoglobin

Standard glycosylated Hb ($HbA_{1c}$) was prepared from pooled heparinized whole blood by catio-exchange chromatography (L. A. Trivelli et al., New England J. of Medicine *284* (1971), 353), using Bio-Rex 70 resin (Biorad, Richmond, Ca, USA). The purified $HbA_{1c}$ was then used to prepare standard samples by recombining it in varying known amounts with blood free from the glycosylated hemoglobin. The concentrations of $HbA_{1c}$ relative to total hemoglobin in the samples varied from 1 to 20% by weight and the total Hb concentration was of the order of 150 g/l.

An analytical apparatus with cuvettes involving a dual waveguide as illustrated on Fig. 2 was used for the determinations; the inner surface of one side of the cuvette was plated with antibody specific to $HbA_{1c}$ while the surface of the opposite side was left free. The content of each cell (a fresh one was used for successively testing each standard) was about 1 ml and 0.1 ml of the standard to be measured with about 0.9 ml of PBS were pipetted therein. Fig. 8 depicts one of the titration curves obtained after 15 min incubation time (with the 20% $HbA_{1c}$ sample), the upper curve (nearly flat) being that recorded with the uncoated part of the guide and the lower curve showing the response of the antibody coated part of the waveguide.

The results of the analysis of the various standards are also gathered in the table below.

| Standard sol. under test (% $HbA_{1c}$) | (%) Transmission in waveguide | | Difference M (%) |
|---|---|---|---|
| | Uncoated side | Coated side | |
| 0 | 56.1 | 55.8 | . 0.3 |
| 1 | 55.5 | 54.7 | 0.8 |
| 5 | 53.7 | 48.2 | 5.5 |
| 10 | 58.0 | 49.2 | 8.8 |
| 20 | 54.9 | 42.4 | 12.5 |

The difference of 0.3% for the zero $HbA_{1c}$ sample indicates some degree of residual affinity of the $HbA_{1c}$ specific antibody for the deglycosylated blood medium. This factor is however considered negligible under practical analytical conditions.

It should also be noted that the % transmission in the uncoated part of the waveguide was not constant from one cell to the other seeming to indicate that the method is not suitable for accurately determining total Hb. However it is not necessary in this instance to measure total Hb, but only to relate the signals from the uncoated and coated sides. Secondly, it is difficult to maintain a degree of constancy in manually fabricating a series of cuvettes such that each will enable full reproducibility of absolute measurements without initial calibration of the equipment. Undoubtedly, when cuvettes are manufactured industrially by molding on a large scale, this disadvantage is overcome.

**Claims**

1. Test probe for optically performing solution analysis including a cuvette for holding a solution to be analyzed and light guiding means comprising a waveguide (50, 70, 90) to be contacted with this solution, some interaction between the evanescent component of a light signal travelling through said waveguide and species in said solution to be analyzed providing desired analytical data, characterized in that said waveguide comprises at least two operatively independent waveguide elements (51, 71, 91; 52, 72, 92) each of which with its own optical signal.

2. Test probe according to claim 1, characterized in that said independent waveguide elements (51, 71, 91; 52, 72, 92) consist of at least two oppositely facing walls of said cuvette (50, 70, 90), said walls being made of materials having identical or different refractive indexes.

3. Test probe according to claim 2, characterized in that the thickness of this cuvette, i.e. the distance between said walls operating as said independent waveguide elements, is of the order of 10 to 1000 μm, the cuvette being then able to be also used as a sampling device due to the pumping capillary effect toward the analyte solution resulting from having the said walls very close to each other.

4. Apparatus including the probe of claims 1, 2 or 3 for the combined determination by optical means of at least two parameters in said solution, said interaction being either with the bulk of the solution or with a complex resulting from the binding of said species to be determined in said solution by reactants specific thereto attached to a waveguide element (51, 71, 91; 52, 72, 92), this apparatus including:

a) light source means (6, 73, 93, 94) for generating at least one light beam and injecting it into a suitable input end of said waveguide;

b) light detecting means (62, 78, 97) for collecting light signals exiting from an outlet of said waveguide and generating electric signals representative of said interaction;

c) computing means (12, 13, 14, 15) for processing said electric signals and providing suitable readouts on said desired determination, wherein each said specific reactant involves one of said independent elements of the waveguide.

5. Apparatus according to claim 4, characterized in that at least one of said waveguide elements (51, 71, 91; 52, 72, 92) is either bare or rendered inhibitory to the binding of said species by means of a blocking agent, said uncoated or blocked element being responsive to said bulk of the solution.

6. Apparatus according to claim 4, in which said light source means (6, 73, 93, 94) comprises chopper means (57, 76) to alternatively inject the signal light into the two elements of the dual-waveguide.

7. Apparatus according to claim 6, in which said chopper means are either a rotating mirror (57) or a chopper disc (76).

8. Apparatus according to claim 4, in which said light source means comprise two independent alternately flashing light sources (93, 94) the output of which is focused each on one optical end of said waveguide elements (91b, 92b) via beam splitting means (98) and in which the other end of said waveguide elements (91a, 91b) is made totally reflective so that the light signal carried by said waveguide elements travels forwards and backwards therein.

**Patentansprüche**

1. Meßfühler zur optischen Bestimmung von gelösten Substanzen, mit einer Küvette zur Aufnahme einer zu analysierenden Lösung und Lichtleitmitteln, die einen Wellenleiter (50, 70, 90) umfassen, der dazu bestimmt ist, in Kontakt mit der Lösung gebracht zu werden, wobei eine gewisse Wechselwirkung zwischen der durch den Wellenleiter hindurchwandernden Dämpfungskomponente eines Lichtsignals und bestimmten Anteilen bzw. Spezien in der zu analysierenden Lösung erwünschte Analysedaten liefert, dadurch gekennzeichnet, daß der Wellenleiter wenigstens zwei wirkungsmäßig unabhängige Wellenleiter - Elemente (51, 71, 91; 52, 72, 92) mit jeweils eigenem optischen Signal umfaßt.

2. Meßfühler nach Anspruch 1, dadurch gekennzeichnet, daß die unabhängigen Wellenleiter - Elemente (51, 71, 91; 52, 72, 92) aus wenigstens zwei einander gegenüberstehenden Wänden der Küvette (50, 70, 90) bestehen, die aus Materialien mit identischen oder unterschiedlichen Brechungsindizes hergestellt sind.

3. Meßfühler nach Anspruch 2, dadurch gekennzeichnet, daß die Dicke dieser Küvette, d.h. der Abstand zwischen den als die unabhängigen Wellenleiter - Elemente wirkenden Wänden in der Größenordnung von 10 bis 1000 μm liegt, was die Küvette auch als Probenehmer verwendbar macht, aufgrund des Pumpen - Kapillareffekts mit Bezug auf die Analyselösung, der darauf beruht, daß die Wände sehr nahe beieinander liegen.

4. Vorrichtung mit dem Meßfühler entsprechend den Ansprüchen 1, 2 oder 3 für die kombinierte optische Bestimmung von wenigstens zwei Parametern in der Lösung, wobei die Wechselwirkung entweder mit der Hauptmenge der Lösung oder mit einem Komplex stattfindet, der aus dem Binden der in der Lösung zu bestimmenden Spezies mittels diesen gegenüber spezifischen Reaktionsteilnehmern resultiert, die an einem Wellenleiter - Element (51, 71, 91; 52, 72, 92) angebracht sind, mit:

8

a) Lichtquellenmitteln (6, 73, 93, 94) zum Erzeugen wenigstens eines Lichtstrahls sowie zu dessen Einleiten in ein geeignetes Einlaßende des Wellenleiters;

b) Lichterfassungsmitteln (62, 78, 97) zum Auffangen von Lichtsignalen, die aus einem Auslaßende des Wellenleiters austreten und zum Erzeugen von die Wechselwirkung repräsentierenden elektrischen Signalen;

c) Rechnermitteln (12, 13, 14, 15) zum Verarbeiten der elektrischen Signale und zum Liefern geeigneter Auslesewerte der gewünschten Bestimmung.

wobei jeder spezifische Reaktionsteilnehmer einem der unabhängigen Elemente des Wellenleiters zugeordnet ist.

5. Vorrichtung nach Anspruch 4, dadurch gekennzeichnet, daß wenigstens eines der Wellenleiter - Elemente (51, 71, 91; 52, 72, 92) entweder freiliegt oder bezüglich des Bindens der Spezien mittels eines Blockieragents hemmend ausgebildet ist, wobei das unbedeckte oder blockierend gemachte Element auf die Hauptmenge der Lösung anspricht.

6. Vorrichtung nach Anspruch 4, bei welcher die Lichtquellenmittel (6, 73, 93, 94) Zerhackermittel (57, 76) zum aufeinanderfolgenden Einleiten des Signallichts in die beiden Elemente des Doppel - Wellenleiters umfaßt.

7. Vorrichtung nach Anspruch 6, bei welcher die Zerhackermittel entweder ein rotierender Spiegel (57) oder eine Zerhackerscheibe (76) sind.

8. Vorrichtung nach Anspruch 4, bei welcher die Lichtquellen mittel zwei unabhängig aufeinaderfolgend blitzende Lichtquellen (93, 94) umfassen, deren jeweilige Wirkleitsungsabgabe über Strahlenteilungsmittel auf ein optisches Ende des Wellenleiter - Elements (91b, 92b) fokussiert ist, und bei welcher das andere Ende des Wellenleiterelements (91a, 91b) totalreflektierend ausgelegt ist, so daß das in den Wellenleiter - Elementen geführte Lichtsignal in diesen vor- und zurückwandert.

## Revendications

1. Sonde d'analyse pour l'analyse optique de substances en solution, laquelle comporte une cuvette contenant la solution à analyser et des moyens de conduction de la lumière comprenant un guide d'onde (50, 70, 90) que l'on met en contact avec cette solution, les mesures analytiques désirées résultant d'une interaction entre les substances de ladite solution d'analyse et le composant d'onde évanescent d'un signal lumineux parcourant ledit guide d'onde, caractérisée en ce que ledit guide d'onde comprend au moins deux éléments guide d'onde indépendants (51, 71, 91; 52, 72, 92) fonctionnant chacun avec son propre signal optique.

2. Sonde analytique suivant la revendication 1, caractérisée en ce que lesdits éléments guide d'onde indépendants (51, 71, 91; 52, 72, 92) sont constitués par au moins deux parois se faisant face de ladite cuvette (50, 70, 90), ces parois étant faites de matériaux présentant un indice de réfraction semblable ou différent.

3. Sonde analytique suivant la revendication 2, caractérisée en ce que l'épaisseur de cette cuvette, c'est-à-dire la distance entre lesdites parois qui fonctionnent comme éléments guide d'onde indépendants, est de l'ordre de 10 à 1000 μm, la cuvette pouvant ainsi être également utilisée comme dispositif d'échantillonnage de la solution de par son effet d'aspiration par capillarité, cet effet provenant de ce que lesdites parois sont très près l'une de l'autre.

4. Appareil incluant la sonde des revendications 1, 2 ou 3 pour la mesure optique combinée d'au moins deux paramètres propres à cette solution, ladite interaction se faisant, soit avec la masse de la solution, soit avec un complexe qui se forme entre lesdites substances à analyser dans la solution et des réactifs qui leur sont spécifiques immobilisés sur un élément guide d'onde (51, 71, 91; 52, 72, 92), cet appareil comprenant:

a) des moyens lumineux (6, 73, 93, 94) pour engendrer au moins un faisceau lumineux et injecter celui-ci à une extrémité d'entrée convenable dudit guide d'onde;

b) des moyens de détection de la lumière (62, 78, 97) pour recueillir les signaux lumineux provenant d'une sortie dudit guide d'onde et pour engendrer un signal électrique caractéristique de ladite interaction;

c) des moyens ordinateurs (12, 13, 14, 15) pour traiter ces signaux électriques et fournir des données de mesure concernant ladite analyse désirée;

caractérisé en ce que chaque réactif spécifique est associé à l'un desdits éléments indépendants du guide d'onde.

5. Appareil suivant la revendication 4, caractérisé en ce qu'au moins un de ces éléments guide d'onde indépendants (51, 71, 91; 52, 72, 92) est, soit dépourvu d'agent réactif, soit rendu non réactif vis-à-vis desdites substances à analyser par la présence d'un agent bloquant ledit élément bloqué ou non réactif engendrant alors un signal au contact de la masse de la solution.

6. Appareil suivant la revendication 4, das lequel les moyens lumineux (6, 73, 93, 94) comportent des moyens de hâchage de la lumière (57, 76) pour injecter le signal lumineux en alternance dans les deux éléments du double guide d'onde.

7. Appareil suivant la revendication 6, dans lequel ces moyens de hâchage consistent en un miroir tournant (57) ou en un disque de hâchage (76).

8. Appareil suivant la revendication 4, dans lequel lesdits moyens lumineux comportent deux lampes éclair fonctionnant en alternance (93, 94) dont l'énergie est centrée chacune sur l'une des extrémités optiques d'un élément guide d'onde (91b, 92b) par l'intermédiaire de moyens optiques de division (98), et dans lequel on a rendu totalement réfléchissante l'autre extrémité desdits éléments guide d'onde (91a, 91b), de manière à ce que le signal lumineux qui parcourt lesdits éléments guide d'onde voyage en avant et en arrière dans ceux-ci.

FIG. 1

FIG. 2

FIG. 3

FIG. 4

FIG. 5

FIG. 6

FIG. 7

FIG. 8